(19) Europäisches Patentamt
European Patent Office
Office européen des brevets

(11) **EP 2 820 962 A1**

(12) **EUROPEAN PATENT APPLICATION**
published in accordance with Art. 153(4) EPC

(43) Date of publication:
**07.01.2015 Bulletin 2015/02**

(51) Int Cl.:
***A23L 3/16*** (2006.01)   ***A23C 3/037*** (2006.01)
***A23L 3/24*** (2006.01)

(21) Application number: **13754555.4**

(22) Date of filing: **01.03.2013**

(86) International application number:
**PCT/JP2013/055699**

(87) International publication number:
**WO 2013/129654 (06.09.2013 Gazette 2013/36)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **02.03.2012 JP 2012046496**

(71) Applicant: **MEIJI CO., LTD**
**Koto-ku**
**Tokyo 136-8908 (JP)**

(72) Inventors:
• **OSADA, Takashi**
  **Odawara-shi**
  **Kanagawa 250-0862 (JP)**
• **MATSUBARA, Hiroki**
  **Odawara-shi**
  **Kanagawa 250-0862 (JP)**

(74) Representative: **Isarpatent**
**Patent- und Rechtsanwälte**
**Friedrichstrasse 31**
**80801 München (DE)**

(54) **STERILIZATION METHOD**

(57)    It is intended to provide a sterilization method for heat- resistant bacterial spores, with which an effective sterilization effect is attained under heating conditions milder than conventional conditions, and which hardly affects the quality of an object to be sterilized. The sterilization method includes performing a first heat treatment step having the main object of damaging spores and then performing a second heat treatment step having the main object of inactivating the damaged spores and being performed at a temperature lower than that of the first heat treatment step.

[Fig. 1]

## Description

Technical Field

The present invention relates to a sterilization method. More specifically, the present invention relates to a method for sterilizing an object to be sterilized which contains bacterial spores and the like having high heat resistance.

Background Art

As a sterilization method in food industries, an effect of killing microorganisms caused by heating (thermal action) is utilized in most cases. Since the bacteria that form spores are hard-to-kill as compared to ordinary microorganisms, heating (sterilization) conditions are necessarily made severer by raising a sterilization temperature as in a UHT treatment (heat treatment at 120°C to 150°C for 1 to 3 seconds) and a HTST treatment (at 72°C to 75°C for 15 seconds or more) or extending a heating and retention time as in an LTLT treatment (heat treatment at 63°C to 65°C for 30 minutes).

However, the severer heating conditions described above entail problems of deteriorations of useful nutritious ingredients (proteins, vitamins, etc.), promotion of generation of components which are the causes of deteriorations of flavor and quality (heating odor such as furosine, etc.), and so forth. Under the circumstances, there has been a demand for development of a method for effectively sterilizing especially the bacteria which form spores among bacterial microorganisms and the bacterial spores by employing milder heating conditions (sterilization temperature and heating and retention time).

For instance, JP 06-303899 A (Patent Literature 1) discloses a method for sterilizing liquid products, characterized by: preliminarily heating a liquid product obtained by using milk or the like as a raw material to a temperature of 90°C to 125°C by indirect heating; performing heating to 140°C to 150°C by spraying steam directly to the product; cooling a temperature of the product to 90°C to 120°C by an indirect cooling step, and cooling to 70°C to 85°C by flash cooling. Since the direct UHT treatment by steam, the rapid indirect cooling, and the flash cooling are performed in this order in the method, the method is capable of suppressing quality deterioration as compared to conventional methods of starting the flash cooling from the high temperature of 140°C to 150°C.

JP 2003-125701 A (Patent Literature 2) discloses a method for sterilizing a fermented milk raw material, characterized by: performing a first heat treatment of heating to a predetermined temperature of 90°C or more and 100°C or less and retaining the temperature for 5 minutes or more; and performing a second heat treatment of heating to a predetermined temperature of 110°C or more and 115°C or less and retaining the temperature for 2 seconds or more and 15 seconds or less, followed by cooling. Since the first heat treatment is performed at 90°C or more and 100°C or less, it is possible to perform the sterilization of spore-forming bacteria at the temperature lower than the heating temperature of the UHT treatment. Therefore, the method is capable of realizing commercialization of fermented milk having favorable flavor attained by long-term fermentation and, in short time fermentation and production of liquid fermented milk and the like, of preventing unfavorable flavor with an alleviated work load of method control of the fermented milk raw material.

JP 05-227925 A (Patent Literature 3) discloses a method for sterilizing bacterial endospores including performing an germination activation treatment by heating bacterial endospores which are an object to be sterilized at 60°C to 100°C and subsequently performing a high pressure treatment at 100 to 1000 MPa and at a room temperature to 100°C for several hours or less. With the method, the sterilization at milder conditions of the shorter treatment time and the lower pressure is enabled as compared to sterilization achieved solely by a high pressure treatment. Also, the publication discloses the sterilization of the bacterial endospores which can be problematic in terms of hygiene and quality control in foods, drugs, and the like by performing heat treatment at 100°C or less.

JP 2003-235528 A (Patent Literature 4) discloses a method for sterilizing foods, characterized by reducing resistance of bacterial spores through a treatment of foods with high pressure carbon dioxide and then performing a sterilization treatment. Since the sterilization is performed at 100°C or less with the method, the bacterial spores in the foods are killed while minimizing quality deterioration of foods caused by heating.

Citation List

Patent Literature

    Patent Literature 1: JP 06-303899 A
    Patent Literature 2: JP 2003-125701 A

Patent Literature 3: JP 05-227925 A
Patent Literature 4: JP 2003-235528 A

Summary of Invention

Technical Problem

However, it cannot be said that the heating conditions during the sterilization are satisfactorily alleviated in the method disclosed in Patent Literature 1 since the endospore-forming bacteria are sterilized by the UHT treatment including direct heating. Further, a generation/increase rate of the flavor/quality-deteriorating component such as furosine is increased in proportion to a heating temperature. Therefore, the method disclosed in Patent Literature 2 in which the temperature in the second heat treatment is relatively high the flavor/quality-deteriorating component tends to be increased.

It is assumed that an amount of the flavor/quality- deteriorating component to be generated is suppressed in the method disclosed in Patent Literature 3 or Patent Literature 4 since the heating is performed at 100°C or less, but the method requires a pressurizing device for retaining the sterilization object under the high pressure. Therefore, not only the facility investment for the pressurizing device, but also a complicated control requiring a third factor of pressure control in addition to the two factors of temperate and time is necessitated. Further, since the high pressure treatment makes it difficult to adopt a continuous method, work efficiency is deteriorated.

An object of the present invention is to provide an effective bacterial spore sterilization method capable of attaining an effect of effectively sterilizing bacteria which form spores having high heat resistance under milder heating conditions than the UHT treatment and the LTLT treatment which are standard sterilization methods and of suppressing degeneration of various useful nutritious ingredients (proteins, vitamins, etc.) and generation/increase of components which are the causes of deteriorations of flavor and quality (heating odor of furosine, etc.) caused by excessive heating (superheating) or the like. Particularly, the object is to provide a sterilization method which is controlled with two factors of a temperature and a time as in the UHT treatment and the LTLT treatment which are the standard sterilization methods.

Solution to Problem

The inventors conducted researches on a sterilization method capable of attaining a favorable effect on bacteria which form spores under mild conditions to find that the bacterial spores are effectively sterilized under milder conditions (sterilization temperature and heating and retention time) than standard sterilization methods, i.e. with a milder thermal history than the standard sterilization methods, by performing a stepwise heat (sterilization) treatment by changing heating conditions, in which heat resistance (heat stability) of the bacterial spores is reduced, and then a heat treatment at a temperature lower than that set for the heat resistance reduction is performed.

Specifically, the invention set forth in claim 1 according to the present invention is a sterilization method characterized by comprising performing a first heat treatment step on an object to be sterilized and then performing a second heat treatment step which is performed at a temperature lower than that of the first heat treatment step.

The invention set forth in claim 2 is the sterilization method according to claim 1, characterized by comprising a preheating step before the first heat treatment step.

The invention set forth in claim 3 is the sterilization method according to claim 1 or 2, characterized by comprising a cooling step after the first heat treatment.

The invention set forth in claim 4 is the sterilization method according to any one of claims 1 to 3, characterized in that the cooling step after the first heat treatment step is performed at a temperature lower than the heat treatment temperature of the second heat treatment step.

The invention set forth in claim 5 is the sterilization method according to any one of claims 1 to 4, characterized in that the first heat treatment step is performed under heating conditions which are not capable of completely inactivating spore-forming bacteria and bacterial spores in the sterilization object solely by the first heat treatment step.

The invention set forth in claim 6 is the sterilization method according to any one of claims 1 to 4, characterized in that the first heat treatment step is performed under heating conditions which are capable of completely inactivating spore-forming bacteria and bacterial spores in the sterilization object solely by the first heat treatment step.

The invention set forth in claim 7 is the sterilization method according to any one of claims 1 to 6, wherein the first heat treatment step is the step in which a temperature of the sterilization object is retained at 100°C to 180°C, and a retention time at the temperature is 1 second or more and 60 seconds or less.

The invention set forth in claim 8 is the sterilization method according to any one of claims 1 to 7, wherein the first heat treatment step is the step in which a temperature of the sterilization object is retained at 100°C to 180°C, and a retention time at the temperature is 1 second or more and 60 seconds or less, and the second heat treatment step is the step in which the temperature of the sterilization object is retained at 40°C to 100°C, and a retention time at the temperature is 5 seconds or more and 60 minutes or less. Advantageous Effects of Invention

Since the present invention combines the first heat treatment step and the second heat treatment step in which the heat treatment is performed at the temperature lower than that of the first heat treatment step, a thermal history accompanying the heat treatments is reduced as compared to a case of attaining a sterilization effect equivalent to the present invention. Therefore, the bacterial spores are inactivated while suppressing deterioration of various useful nutritious ingredients (proteins, vitamins, etc.) and generation/increase of components which are the causes of deteriorations of flavor and quality (heating odor of furosine, etc.). More specifically, the treatment is performed in the first heat treatment step under the heating conditions which are not capable of completely inactivating the spore-forming bacteria and bacterial spores, e.g. under the heating conditions capable of damaging the spores, and then the damaged spores are inactivated when treated under the heating conditions including the temperature lower than that of the first heat treatment step. In other words, in the case where a microorganism-killing effect to be attained when the first heat treatment step and the second heat treatment step are finished is set to the effect equivalent (similar) to those of the standard methods, foods and beverages (fruit juice, milk, dairy product, soymilk, soy product (e.g. soybean curd)) with higher freshness and richer flavor are produced with the milder thermal history.

Alternatively, the first heat treatment may be performed at heating conditions which are capable of completely inactivating the spore-forming bacteria and bacterial spores, e.g. under heating conditions of the standard UHT treatment, and then the second heat treatment may be performed under heating conditions including a temperature lower than that of the first heat treatment step. In other words, in the case where the thermal history is set to that equivalent (similar) to or more than those of the standard methods, the sterilization method attains a higher microorganisms-killing effect than the conventional methods and reduces the danger (risk) of bacterial contamination, thereby extending a shelf life.

Further, it is possible to put the present invention into practice by adding a small change to an existing treatment apparatus since the heat treatments are performed without application of a high pressure.

Brief Description of Drawings

Fig. 1 is a graph showing one example of a relationship between a time and a temperature in the sterilization method of the present invention.
Fig. 2 is a block flow diagram showing the example of the present invention.
Fig. 3 is a graph showing a temperature profile of the embodiment of the present invention.
Fig. 4 is a graph showing results of measurements of numbers of cells of Bacillus cereus B-164 spores according to the embodiment of the present invention.

Description of Embodiments

A sterilization method of the present invention comprises a first heat treatment step which is performed on an object to be sterilized and a second heat treatment step which is performed after the first heat treatment and at a temperature lower than that of the first heat treatment step. When so required, the sterilization method comprises a preheating step which is performed before the first heat treatment step. Further, the sterilization method comprises a cooling step which is performed after the first heat treatment step as required.

The preheating step is the step for heating the sterilization object which has been stored in a refrigerator or at a room temperature until a temperature of the sterilization object reaches to a predetermined temperature of 40°C to 100°C. The preheating step is performed for the purpose same as that of a preheating step performed in known sterilization methods such as a UHT treatment and a HTST treatment.

A heating device used for the preheating step may be any device insofar as the device is capable of heating from 1°C to 30°C to about 40°C to 100°C. A heating method is not particularly limited, and examples thereof include indirect heating, direct heating, electric heating, microwave heating, and the like.

The preheating step is not essential and can be omitted as required. In short, the method of the present invention may be started from the first heating step.

A temperature condition of the preheating step may preferably be 50°C to 95°C, more preferably 60°C to 90°C, furthermore preferably 70°C to 85°C, and particularly preferably 75°C to 85°C, though it is varied depending on the temperature in the first heat treatment step.

The sterilization method of the present invention comprises the first heat treatment step which may be performed after the preheating step. The first heat treatment step is the step for heating the sterilization object to a high temperature and retaining the temperature for a short time. The step is a first stage of inactivating bacteria and spores (also called endospores) in the sterilization object. In the step, either one of heating conditions which are not capable of completely inactivate the spore-forming bacteria and the spores solely by the step or heating conditions which are capable of completely inactivating the spore-forming bacteria and the spores may be selected.

More specifically, in the case where it is desired to attain a microorganism sterilization effect equivalent to that of the standard methods with a mild thermal history, the heating conditions under which the spore-forming bacteria and bacterial spores are not completely inactivated solely by the step may be selected. Alternatively, in the case where it is desired to attain a microorganism sterilization effect equivalent to or more than that of the standard methods, the heating conditions under which the spore-forming bacteria and bacterial spores are completely inactivated solely by the step may be selected. In other words, the aim of the first heat treatment step is to at least cause damage to the bacterial spores. Preferably, the heating conditions with the aim of sterilizing general bacteria and causing damage to the bacterial spores may be set in order to attain the mild thermal history, and the heating conditions with the aim of the completely inactivation of spore-forming bacteria and spores may be set in order to alleviate the risk of bacterial contamination. The thermal history is an index which is understood in proportion to the product of the heating temperature and the temperature retention time in the sterilization step, and it is considered that the thermal history is increased along with increases in heating temperature and temperature retention time. Also, as used herein, the heating conditions under which are not capable completely inactivation of spore forming bacterial and spores mean the conditions which are milder than those of the UHT treatment and the HTST treatment which are the standard sterilization methods and are not capable of attain the inactivation of bacterial spores solely by the step, for example, and it is not questioned as to whether or not the general bacteria are killed by the heating conditions.

More specifically, the sterilization object is heated to a predetermined temperature of 100°C to 180°C and retained at the temperature for a time period of 1 second or more and 60 seconds or less in the step. A heating device to be used for the first heat treatment step may be any heating device insofar as the device is capable of heating from about 40°C to 100°C to 100°C to 180°C. A heating method is not particularly limited, and examples thereof include indirect heating, direct heating, electric heating, microwave heating, and the like. In the step, a continuous device or equipment such as a plate heat exchanger, a tube heat exchanger, a steam injection heater, a steam infusion heater, and a joule heating type heater may be used. Also, a batch type device or equipment such as a tank or a tray may be used.

For example, the heating conditions may be 110°C to 160°C for a retention time of 1 to 30 seconds, 110°C to 150°C for a retention time of 1 to 20 seconds, 115°C to 145°C for a retention time of 1 to 15 seconds, 120°C to 145°C for a retention time of 1 to 10 seconds, 100°C to 120°C for a retention time of 1 to 10 seconds, 100°C to 115°C for a retention time of 1 to 10 seconds, or 110°C to 115°C for a retention time of 1 to 5 seconds. Among these conditions, the preferred conditions are 100°C to 120°C for a retention time of 1 to 10 seconds, and more preferred conditions are 110°C to 115°C for a retention time of 1 to 5 seconds, in order to attain the mild thermal history. Further, in order to attain the microorganism sterilization effect equivalent to or more than those of the standard methods, the preferred conditions are 115°C to 145°C for a retention time of 1 to 15 seconds, and the more preferred conditions are 120°C to 145°C for a retention time of 1 to 10 seconds. More specifically, in order to attain the latter effect, the heating temperature and the temperature retention time in the ultrahigh temperature (UHT) sterilization method or the like, for example, may be selected.

The sterilization method of the present invention comprises a cooling step which is performed after the first heat treatment step as required. The cooling step is the step for reducing the temperature of the sterilization object which underwent the treatment with the high temperature. For the reason in terms of equipment or the reason of facilitating inactivation of the spores which are not damaged by the first heat treatment step, and the like, the cooling step may be comprised in order to temporarily reduce the product temperature before entering the second heat treatment step. In the cooling step, the sterilization object is cooled to a predetermined temperature of 80°C to 1°C, preferably to a temperature lower than a temperature retained in the second heat treatment step. A cooling device to be used in the cooling step may be any cooling device insofar as the device is capable of cooling from about 100°C to 180°C to about 80°C to 1°C. A cooling method is not particularly limited, and examples thereof include indirect cooling, vacuum cooling (rapid decompression boiling and cooling), a method of directly injecting cooling water, and the like, for example.

In the step, the sterilization object may preferably be cooled to 70°C to 1°C, more preferably 60°C to 1°C, furthermore preferably 50°C to 1°C, and particularly preferably 40°C to 1°C. Because the step is the arbitrarily selected step, the method may proceed to the second heat treatment step immediately after the first heat treatment is finished.

The sterilization method of the present invention comprises the second heat treatment step. The second heat treatment step is the step for heating the sterilization object, retaining the temperature, and cooling as required. The temperature retained in the second heat treatment step is lower than the temperature of the first heat treatment step. In the step, the treatment in the first heat treatment step is reinforced. More specifically, the second heat treatment step is performed with the aim of sterilizing general bacteria and inactivating bacterial spores and inactivates the bacterial spores that are not completely inactivated by the first heat treatment step. Also, the second heat treatment step sterilizes the general bacteria which are not sterilized by the first heat treatment step. Since the bacterial spores and the like having high heat resistance contained in the sterilization object are damaged by the first heat treatment step, it is unnecessary to newly apply a large thermal load (high temperature and long time) in order to sterilize the bacterial spores, and a satisfactory sterilization effect is attained at a low temperature and a short time. Also, by performing the heat treatment at the low temperature, the bacterial spores and the like that are not inactivated by the first heat treatment step are inactivated without fail. Further, even in the case where the bacterial spores and the like are completely inactivated in the first heat treatment step, the more reliable inactivation of bacterial spores and the like is achieved, and the risk by bacterial contamination is suppressed.

In the step, more specifically, the sterilization object is heated until the temperature thereof reaches to a predetermined temperature of 40°C to 100°C, and the temperature is retained for a time period of 5 seconds or more and 60 minutes or less. A heating device to be used in the second heat treatment step may be any heating device insofar as the device is capable of heating from about 1°C to 80°C to about 40°C to 100°C. A heating method is not particularly limited, and examples thereof include indirect heating, direct heating, electric heating, microwave heating, and the like. In the step, a continuous device or equipment such as a plate heat exchanger, tube heat exchanger, a steam injection heater, a steam infusion heater, and a joule heating type heater and, also, a batch type device or equipment such as a tank or a tray may be used.

For example, the heat treatment conditions are about 50°C to 100°C for a retention time of 5 seconds to 30 minutes, about 60°C to 100°C for a retention time of 5 seconds to 20 minutes, about 70°C to 100°C for a retention time of 5 seconds to 10 minutes, about 80°C to 100°C for a retention time of 5 seconds to 10 minutes. More specifically, the heating conditions in the low temperature long time (LTLT) sterilization method, the high temperature short time (HTST) sterilization method (72°C to 75°C for 15 seconds or more), or the like may be adopted.

A temperature for cooling after the heat treatment may be 30°C to 1°C, 20°C to 1°C, 15°C to 1°C, or 10°C to 1°C, for example.

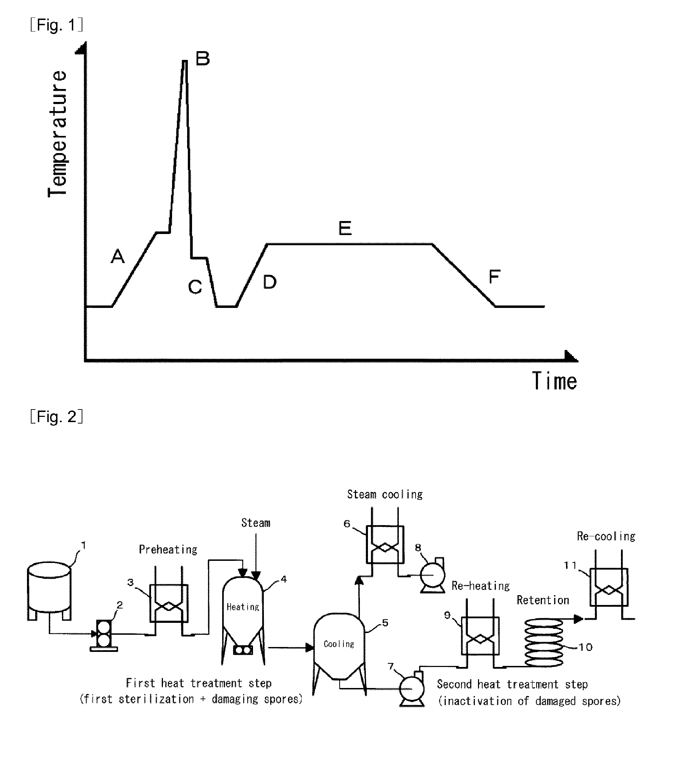

The sterilization method of the present invention comprises the above-described steps. A relationship between the time and the temperature in the sterilization method of the present invention is shown in Fig. 1. In Fig. 1, the section A indicates the preheating step, the section B indicates the first heat treatment step, the section C indicates the cooling step, and the sections from D to F indicate the second heat treatment step.

The sterilization object on which the sterilization method of the present invention is to be performed is not particularly limited insofar as the sterilization object can be sterilized by heating and may preferably be a substance which can cause a hygienic problem due to bacterial contamination and is subject to change in quality due to heating. For example, the sterilization object may be a beverage or a food, more specifically, a fruit juice, milk, a dairy product, soymilk, a soybean product (soybean curd), a soft drink such as green tea, or the like. The sterilization object may be a finished product (product to be sterilized), an intermediate product in the middle of manufacture, or a raw material.

Hereinafter, the present invention will be described in more details based on one example. It should be understood that the example does not limit the present invention.

Example 1

A sterilization experiment using bacterial spores was conducted. Heat resistance information about the bacterial spores is shown in Table 1. As used herein, the D-value means a time required for reducing the number of cells to 1/10, i.e. for killing 90% of the cells, by heating a bacterium and endospores in certain amounts to a certain temperature. The Z-value means a temperature variation width which reduces the D-value to 1/10 or increases the D-value by a factor of 10 (Kishimoto, "Retort Foods", Kabushiki Kaisha Korin, pp. 37 to 38, 1994).

[Table 1]

| Name of Bacteria and Bacteria No. | Z-value | D-value (D110) | D-value (D120) |
|---|---|---|---|
| | [°C] | [sec] | [sec] |
| Bacillus cereus | | | |
| B-164 spores | 9.4 | 132 | 11.2 |

Bacillus cereus B-164 (derived from raw milk) was cultured at 30°C overnight in a tripticase soy broth and then was coated on a Schaeffer's sporulation medium agar (DSM, Difco sporulation medium), followed by culturing at 30°C for 7 days. After the culturing, sterilized ion exchange water was added, and a colony on a surface of the culture medium was scraped off with a sterilized cotton swab and then was placed in a centrifugal tube, followed by bacterium collection by centrifugation (3500 rpm for 15 minutes). Subsequently, centrifugation (3500 rpm for 15 minutes) using sterilized ion exchange water was performed for 3 times, and sterilized ion exchange water was added again to cause heat shock (80°C for 10 minutes) to kill the nutritive cells. The bacterial liquid after the treatment was immediately cooled to be used as a bacterial spore suspension liquid.

The thus-prepared bacterial spore suspension liquid was subjected to sterilization in accordance with the block flow shown in Fig. 2. The bacterial spore suspension liquid was poured into a balance tank 1 and fed to a sterilization device (SPP type sterilizer manufactured by SPX Corporation) at a predetermined feed rate (100 L/hr) via a pump 2. As the preheating step, the bacterial spore suspension liquid of 20°C was heated (preheated) to 70°C by using a preheating plate 3. After that, as the first heat treatment step, the bacterial spore suspension liquid was fed to a heating device (steam infusion chamber) 4 and was instantaneously heated to 110°C. After retaining the temperature for 3 minutes, rapid cooling to 64°C was conducted in a vacuum evaporation cooling tank 5. Subsequently, as the second heat treatment step, the bacterial spore suspension liquid was heated again to 80°C to inactivate the damaged spores using a heating plate 9 and was retained at 80°C for 10 minutes using a temperature retention coil 10. After that, cooling to 30°C was conducted by using a cooling plate 11. In Fig. 2, a pump 7 feeds the bacterial spore suspension liquid to the heating plate 9, a cooling device 6 cools the steam generated in the vacuum evaporation cooling tank 5, and a pump 8 feeds cooling water to the cooling device 6.

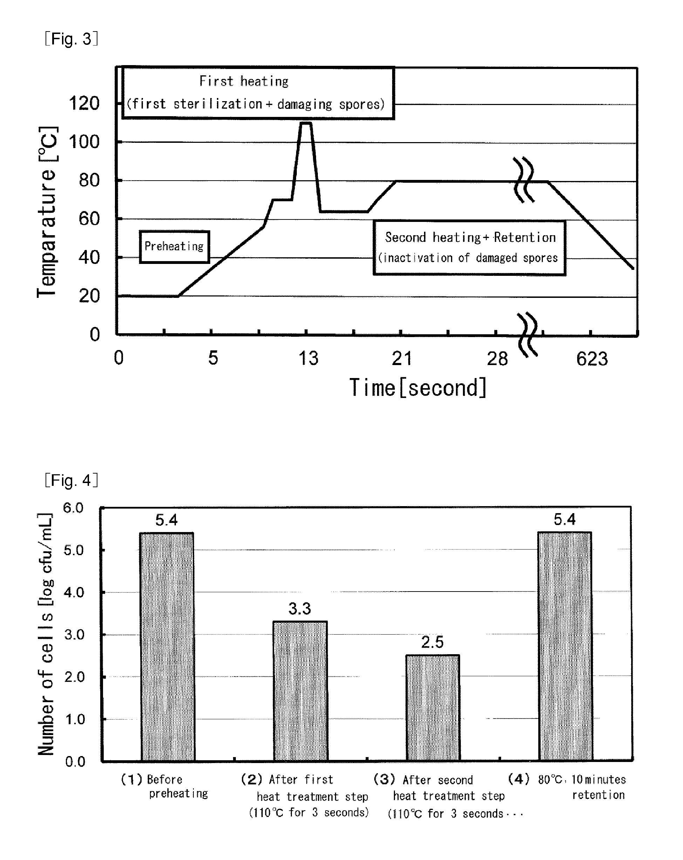

In the present example, sampling was conducted (1) before the preheating step, (2) immediately after the first heat treatment step, and (3) after the second heat treatment step to measure the number of cells. Further, (4) the bacterial spore suspension liquid was separately retained to 80°C for 10 minutes to count the number of cells. The temperature profile of the present example is shown in Fig. 3, and results of measurements of the numbers of cells of B-164 spores are shown in Fig. 4.

From the results shown in Fig. 4, it was confirmed that the number of cells of 5.4 [log cfu/mL] before the preheating step is reduced to 3.3 [log cfu/mL] after the first heat treatment step and is reduced to 2.5 [log cfu/mL] after the second heat treatment step which was performed at the alleviated heating conditions. In view of the D-value of the bacteria (D110 = 132 seconds), the number of cells is not theoretically reduced under the sterilization conditions (80°C for 10 minutes) of the second heat treatment step. Further, as shown in (4) of Fig. 4, any reduction of the bacterium was not observed only by the retention at 80°C for 10 minutes. In short, the effective sterilization effect on the bacterial spores having high heat resistance was attained by combining a damaging effect to the bacterium + spores of the first heat treatment step with the alleviated sterilization conditions of the second heat treatment.

Though the steam was used for the heating in the first heat treatment step in the present example, it is considered that a higher sterilization effect is attained by combining the steam with a method of damaging the bacterial spores by an electrical action (electric pulse sterilization, AC high electric field sterilization, or the like).

Heating conditions required for attaining a sterilization effect equivalent to that of the present invention by one heating operation were calculated, and an amount of each of substances generated by heating under the heating conditions was estimated to compare the estimated value with an amount of each of substances generated by heating under the heating conditions of the present invention (estimated value: total amount of the amounts in the first heat treatment step and the second heat treatment step). The amounts of various heat-generated substances were estimated with reference to the publication (Claeys WL et al., J Dairy Res, 2003, Feb; 7(1), 85-90) and using the following Expression 1.

[Expression 1]

$$C = C_0 + k_{ref} \cdot \exp\left[\frac{E_a}{R} \cdot \left(\frac{1}{T_{ref}} - \frac{1}{T}\right)\right] \cdot t$$

In Expression 1, C indicates a concentration of a heat-generated substance, Co indicates an initial concentration of the heat-generated substance, kref indicates a reaction rate, Ea indicates activation energy [kJ/mol], R indicates a general gas constant [J/(mol·K)], Tref indicates a reference temperature [K], T indicates a treatment temperature [K], and t indicates a treatment time [minute].

In the present example, hydroxymethylfurfural (HMF), lactulose, and furosine were selected as the heat-generated substances to be calculated. Each of the substances is known to be generated as a result of heating milk. Shown in Table 2 are kref, Ea, and Tref of HMF, lactulose, and furosine in milk.

[Table 2]

|  | HMF | Lactulose | Furosine |
|---|---|---|---|
| kref | 0.771 [μmol/L/min] | 31.7 [mg/L] | 9.64 [mg/100 g protein] |
| Ea [kJ/mol] | 116 | 109.2 | 88.4 |

(continued)

|  | HMF | Lactulose | Furosine |
|---|---|---|---|
| Tref [°C] | 105 | 105 | 110 |
| (Source of reference: Claeys WL et al., J Dairy Res, 2003 Feb; 7(1), 85-90) | | | |

From the results obtained by the present example and the values shown in Table 1, it was calculated that, in order to attain a sterilization effect of 2.9 [log cfu/mL] by performing sterilization at 110°C only once, retention of 132 [second/(log cfu/mL)] x 2.9 [log cfu/mL] = 382.8 [seconds] at 110°C is required for attaining the sterilization effect of 2.9 [log cfu/mL]. In the case of 120°C, it was calculated that, in order to attain a sterilization effect of 2.9 [log cfu/mL] by performing sterilization at the temperature only once, retention of 11.2 [second/(log cfu/mL)] x 2.9 [log cfu/mL] = 32.5 [seconds] at the temperature is required for attaining the sterilization effect of 2.9 [log cfu/mL].

Based on the results, an amount of each of the heat-generated substances under the sterilization conditions that enable to attain the sterilization effect of 2.9 [log cfu/mL] by the present invention and an amount of each of the heat-generated substances under the sterilization conditions that enable to attain the sterilization effect of 2.9 [log cfu/mL] by performing the sterilization only once are compared to each other, and the results of the comparison are shown in Table 3.

[Table 3]

| Heating conditions for attaining equivalent sterilization effect (2.9D reduction) | HMF [μmol/L/min] | Lactulose [mg/L] | Furosine [mg/100 g protein] |
|---|---|---|---|
| Present invention (110°C for 3 seconds + 80°C for 10 minutes) | 0.63 | 29.55 | 9.59 |
| Single step sterilization (110°C for 383 seconds) | 8.0 | 318.5 | 61.5 |
| Single step sterilization (120°C for 32.5 seconds) | 1.7 | 64.7 | 10.6 |
| Single step sterilization (110C) ÷ present invention | 12.7 | 10.8 | 6.4 |
| Single step sterilization (120°C) ÷ present invention | 2.7 | 2.2 | 1.1 |

From the results shown in Table 3, it was confirmed that the retention time required for attaining the equivalent sterilization effect at the temperature (110°C) equal to the highest temperature of the sterilization method of the present invention is 383 seconds, and it was estimated that the amounts of the heat-generated substances generated during the retention time are 12.7 times (HMF), 10.8 times (lactulose), and 6.4 times (furosine) larger than those generated in the present invention.

Likewise, it was confirmed that the retention time required for attaining the equivalent sterilization effect at the temperature (120°C) higher than the sterilization method of the present invention is 32.5 seconds, and it was estimated that the amounts of the heat-generated substances generated during the retention time are 2.7 times (HMF), 2.2 times (lactulose), and 1.1 times (furosine) larger than those generated in the present invention.

In view of the above, it was inferred that the sterilization method of the present invention enables to suppress the amounts of heat-generated substances even under the milder conditions than the sterilization conditions that attain the equivalent sterilization effect and, therefore, enables to obtain a product which is fresher and has a favorable flavor. Further, it was inferred that, as compared to the case of performing the heat treatment under the conditions of 110°C for the heating time of 383 seconds or more or the heat treatment under the conditions of 120°C for the heating time of 32.5 seconds or more, the amounts of the heat-generated substances are suppressed and that a product which is fresher and has a favorable flavor is obtained in the case of performing the heat treatment at 110°C or the heat treatment at 120°C and then performing the second heat treatment after the 110°C- or 120°C-heat treatment under the condition that is milder than those of the 110°C- or 120°C-heat treatment.

Industrial Applicability

The present invention enables to develop the effective bacterial spore sterilization method which is capable of suppressing deteriorations of various useful nutritious ingredients (proteins, vitamins, etc.) and generation/increase of components which are the causes of deteriorations of flavor and quality (heating odor of furosine, etc.) caused by excessive heating (superheating) or the like. Therefore, the present invention enables to produce products which are suppressed in quality deterioration due to heating in the field of foods, for example. Also, as compared to the conventional sterilization methods, the present invention enables developments and manufactures of products which are much more suppressed in microbiological risk, such as development and manufacture of a product having a longer shelf life, for example.

**Claims**

1. A sterilization method **characterized by** comprising performing a first heat treatment step on an object to be sterilized and then performing a second heat treatment step which is performed at a temperature lower than that of the first heat treatment step.

2. The sterilization method according to claim 1, **characterized by** comprising a preheating step before the first heat treatment step.

3. The sterilization method according to claim 1 or 2, **characterized by** comprising a cooling step after the first heat treatment.

4. The sterilization method according to any one of claims 1 to 3, **characterized in that** the cooling step after the first heat treatment step is performed at a temperature lower than the heat treatment temperature of the second heat treatment step.

5. The sterilization method according to any one of claims 1 to 4, **characterized in that** the first heat treatment step is performed under heating conditions which are not capable of completely inactivating spore-forming bacteria and bacterial spores in the sterilization object solely by the first heat treatment step.

6. The sterilization method according to any one of claims 1 to 4, **characterized in that** the first heat treatment step is performed under heating conditions which are capable of completely inactivating spore-forming bacteria and bacterial spores in the sterilization object solely by the first heat treatment step.

7. The sterilization method according to any one of claims 1 to 6, wherein the first heat treatment step is the step in which a temperature of the sterilization object is retained at 100°C to 180°C, and a retention time at the temperature is 1 second or more and 60 seconds or less.

8. The sterilization method according to any one of claims 1 to 7, wherein the first heat treatment step is the step in which a temperature of the sterilization object is retained at 100°C to 180°C, and a retention time at the temperature is 1 second or more and 60 seconds or less, and the second heat treatment step is the step in which the temperature of the sterilization object is retained at 40°C to 100°C, and a retention time at the temperature is 5 seconds or more and 60 minutes or less.

[Fig. 1]

[Fig. 2]

[Fig. 3]

[Fig. 4]

Form PCT/ISA/210 (second sheet) (July 2009)

**INTERNATIONAL SEARCH REPORT**

| International application No. |
| --- |
| PCT/JP2013/055699 |

A. CLASSIFICATION OF SUBJECT MATTER
*A23L3/16*(2006.01)i, *A23C3/037*(2006.01)i, *A23L3/24*(2006.01)i

According to International Patent Classification (IPC) or to both national classification and IPC

B. FIELDS SEARCHED

Minimum documentation searched (classification system followed by classification symbols)
A23L3/16, A23C3/037, A23L3/24

Documentation searched other than minimum documentation to the extent that such documents are included in the fields searched

Electronic data base consulted during the international search (name of data base and, where practicable, search terms used)
JSTPlus/JMEDPlus/JST7580(JDreamIII)

C. DOCUMENTS CONSIDERED TO BE RELEVANT

| Category* | Citation of document, with indication, where appropriate, of the relevant passages | Relevant to claim No. |
| --- | --- | --- |
| X/Y | JP 1-202247 A  (Suntory Ltd.),<br>15 August 1989 (15.08.1989),<br>entire text; particularly, claims<br>(Family: none) | 1,3,4,7,8/<br>1-8 |
| Y | JP 6-303899 A  (Tetra Laval Holdings & Finance S.A.),<br>01 November 1994 (01.11.1994),<br>entire text; particularly, claims<br>& US 5443857 A          & EP 617897 A1 | 1-8 |

☐ Further documents are listed in the continuation of Box C.      ☐ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| 01 July, 2013 (01.07.13) | 09 July, 2013 (09.07.13) |

| Name and mailing address of the ISA/<br>   Japanese Patent Office | Authorized officer |
| --- | --- |
| Facsimile No. | Telephone No. |

**EP 2 820 962 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6303899 A
- JP 2003125701 A
- JP 5227925 A
- JP 2003235528 A

**Non-patent literature cited in the description**

- **KISHIMOTO.** Retort Foods. *Kabushiki Kaisha Korin,* 1994, 37-38
- **CLAEYS WL et al.** *J Dairy Res,* February 2003, vol. 7 (1), 85-90